# EUROPEAN PATENT SPECIFICATION

(11) **EP 3 104 815 B1**
(45) Date of publication and mention of the grant of the patent: **09.01.2019**
(21) Application number: 14883534.1
(22) Date of filing: 04.06.2014
(51) Int. Cl.: A61F 13/00, A61N 1/00, A61L 15/32

(54) **BIORESONANT DRESSING FOR RAPID HEALING OF WOUNDS, PROCESS FOR OBTAINING THE SAME AND USE THEREOF**
BIORESONANTER VERBAND ZUR SCHNELLEN HEILUNG VON WUNDEN, VERFAHREN ZUR HERSTELLUNG DAVON UND VERWENDUNG DAVON
PANSEMENT BIO-RÉSONANT POUR CICATRISATION RAPIDE DE PLAIES, PROCÉDÉ POUR L'OBTENIR ET SON UTILISATION

(30) Priority: 03.02.2014 RO 201400085
(43) Date of publication of application: 21.12.2016
(73) Proprietor: Sanimed International Impex Srl, Bucuresti (RO)
(72) Inventor: VELCEA, Marian, Bucuresti (RO); NEGRUTA, Mircea, Campina (RO); HIDEG, Robertino Catalin, Bucuresti (RO)
(74) Representative: Fierascu, Cosmina-Catrinel
(86) International application number: PCT/RO2014/000016
(87) International publication number: WO 2015/174873

(56) References cited:
- EP-A1- 2 127 694
- EP-A2- 0 227 955
- WO-A1-2009/146441
- WO-A2-2011/133539
- WO-A2-2013/074576

## Description

The invention relates to a bioresonant dressing for the rapid healing of the wounds, a process for obtaining the same and the use thereof.

Modern dressings are carried out by using collagen. These drssings have a crosslinked structure similar to that of the skin and the appearance of a small-pored sponge. The indications for the use thereof are for non-infected, minimum secretion superficial and deep wounds, for skin ulcers and bleeding wounds. Collagen constitutes the most common structural protein of the animal and human organism, which defines a family of scleroproteins with different genetic and structural types and with specific physical-chemical and functional properties.

The advantage of the lack of antigenicity, the unique capacity of collagen of self-assembling in vitro under various modellable shapes (Pachence J.H., Berg R.A, Silver F.H. "Collagen: Its place in the medical device industry", Med Device and Diag Ind. 9, 45-55, 1987) justifies its selection for making dressings.

There are known 13 types of collagen (Collagen 1 to Collagen 13). Derma mainly contains Collagen 1 (85%) with the main function of ensuring the resistance to stretching and Collagen 2 (5%) with the main function of ensuring the resistance to intermittent pressure.

By heating the collagen, this is gradually converted from a very well-organized protein in crystalline state into gelatine - a disorganized and denaturated gel. The cause is the breaking of the hydrogen bonds which ensure the maintaining of the triple helical spiral oriented counterclockwise of the three polypeptide chains of collagen. The temperature where half of the dissolved collagen molecules lost the spiral structure is known as the melting temperature (Tm). The denaturation temperature of the collagen of mammalian origin is usually of 65°C.

Other proteins of the extracellular matrix in the conjunctive tissues together with collagen, such as fibronectine, laminine, fibrine are also suggested as possible successful components together with polysaccharides-glycozaminoglycans (chondroitin, heparin, dermatan..sulphate), hyaluronic acid as well as natural vegetal origin substances or synthetic polymers.

There can be mentioned the patents: US 4412947, US 4837285, US 4970298, US 5869080, EP A 0274898, EP A 0562862 as well as the Romanian patents RO 111335 B1 , RO 115694 B1 and RO 126836 AO.Collagen heating may be caused by the exposure to radiofrequency-RF electromagnetic fields. The electromagnetic radiation is traditionally divided into the following spectra: cosmic rays, gamma rays, ultraviolet, visible range, infrared, microwaves, radiofrequency. The term radiofrequency is dedicated to name the portion of the electromagnetic spectrum in which the electromagnetic waves may be generated by applying alternating current to an antenna. The electromagnetic waves are capable of propagating in vacuum unlike the mechanical waves which need a material medium. The electromagnetic radiation has a magnetic field component and an electric field component.

The electromagnetic waves are differentiated according to their frequency. The electromagnetic radiations have two important characteristics: photonic energy and the propagation speed. The propagation speed in vacuum is of 300000 km/s and does not depend on the frequency or power.

Radiofrequency - RF is a type of electromagnetic radiations with the frequency in the range of 1 GHz - 3- KHz, i.e with a wave length in the range 1 m -10km.

Frequencies over 1 GHz belong to the microwave spectrum.

Radiofrequency is usually divided into the following frequency bands: UHF- ultra high frequency (300 MHz - 3000 MHz), VHF - very high frequency (30 -300 MHz), HF - high frequency (3 - 30 MHz), MF - medium frequency (300 KHz- 3 MHz), LF - low frequency (30 - 300 KHz). The frequency bands MF and HF are the most used ones in the aesthetic medicine ((14) Sistemas Electronicos de Communicaciones, p 21-p.23 Frenzel, Louis L. - Mexico D.F.Alfaomega, 2003).

The living tissue response to radiofrequency exposure depends on several factors, among which: the highest temperature reached (peak temperature), time of exposure to radiofrequency and the mechanical stress applied to the tissue during the heating process. The thermal properties of the tissue may also vary depending on species, age, pH, concentration of electrolytes, concentration and orientation of collagen fibres and the humidity levels in the tissue.

The selective thermolysis produced by the radiofrequency depends on the electrical properties of the tissue, in contrast with the exposure to optical visible energy which depends on the amount of skin chromophore for obtaining the selective destruction of the target tissue ((19) "Effect of controlled volumetric Tissue Heating with Radiofrequency on Cellulite and the Subcutaneous Tissue of the Buttocks and Thighs", Journal of Drugs in Dermatology, 2006, 5; 8 : 714 -722, M.Emilia del Pino, MD, Ramon H.Rosado, MD).

By applying radiofrequency to the skin level, the energy waves induce a high speed molecular oscillation and cause a displacement of the charged particles, involving the generation of rapid rotation motions of the water molecules. The thermal energy of the warm water molecules is released to the adjacent tissue depending on the thermal conductivity thereof. When the tissue is therapeutically heated, the produced and better localized blood flows favour the draining of the zones affected by edema and swollen by the retained fluids. ((19)"Effect of Controlled Volumetric Tissue Heating with Radiofrequency on Cellulite and the Subcutaneous Tissue of the Buttocks and Thighs", Journal of Drugs in Dermatology, 2006, 5; 8: 714 - 722). M.Emilia del Pino, MD, Ramon H. Rosado, MD) ((20) "The medical face lift: A non invasive, non surgical approach to tissue tightening in facial skin, using non ablative radiofrequency", Dermatology Surgery, 2003, 29: 325 - 332, Ruiz Esparza).

The radio frequency energy causes a heating of dermis and fat tissue septa without the heating of epidermis. Heating is generated by inducing the rotary motion of the water molecules due to the occurrence of alternating magnetic fields and to the corresponding electromagentic wave as well as to the tissue resistance to conduction. The rotary motion of the water molecules generates a mechanical friction effect which generates heat. This heat causes the tightening of fibrous septa as well as the generation of an effect of temporary vasodilation and drainage from the fat deposits to the lymphatic system and partial induction of adipocytes by thermal effect on the fat cells, since these are extremely thermolabile. The treatment is intended to increase the tissue temperature with a view to reaching a local temperature exceeding 40°C which triggers a cascade of very useful physiologicl reactions, such as the local vasodilation and stimulation of a new collagen (neocollagen) formation.

In reality there exist two types of effects on the collagen:
- an immediate shrinkage of the collagen extant at the moment of treatment;
- a remodelling and renovation carried on on an average term. (It is important for us to underline the fact that the local vasodilation has an effect of direct amelioration of the microcirculation, which improves the exchange capacity as a complementary biological effect).

Neocollagen is produced as a consequence of inducing the HSP (Heat Shock Protein) release. HSPs are always present in the cells, but their presence is intensified in stress conditions, such as: temperature increase, temperature decrease, trauma, ischemia, ionizing radiations, presence of noxius substances, changes of the osmotic pressure or acute pH (for example a temperature increase by 5°C determines such a synthesis increase, so that these proteins reach 20% of the total proteins of the cells). The HSPs function is to help the preservation or to degrade the proteins that are denaturated by the effect of a stress condition, such as heat ((23) "Hsc62, Hsc56 and GrpE, the third Hsp70 chaperone system of Escherichia coli", Biochem. Biophys. Res.Commun. 293: 2002, (5) : 1389 -95. Yoshimune K, Yoshimura T, Nakayama T, Nishino T, Esaki Λ/.)/((24) "A hitchhiker's guide to the human HSP70 family", Cell Stress Chaperones 1 : 1996, (1): 23 - 8. Tavaria M, Gabriele T, Kola I, Anderson RL).

The HSPs that are synthesized in certain stress conditions initiate the process of tissue repair. The HSP-47 formation is particularly induced by radiofrequency. HSP-47 is a protein that hosts the endoplasmatic reticle and identifies the triple spiral of collagen. The molecules of Collagen I are assembled correctly three-dimensionally only in the presence of HSP-47. The presence of heat as sufficient stimulus seemes to release TFG beta-47 (Transforming Growth Factor Beta -1) which is strongly linked to the process of conjunctive tissue repair; practically, it has the role of extracellular substances (collagen) production accelerator which induces the formation HSP-47, which the fibroblasts recognize as a stimulus for initiating the new collagen synthesis ((25) "Expression of HSP 47 in mouse liver". Cell Tissue Rest: 1996, 288: 341-346. awacfa N, Kuroki T, Kowasky K et als)/ (26) Doctoral Thesis. Institute of Biochemistry (CSIC UCM) School of Pharmacy. Carmen Diaz Fernandez y Maria Cascales/(. 27) "Un FGF-2 modificado acelera la cicatrizacion de las heridas".Jose Antonio Andrades Gomez, Profesor Asociado en el Departamento de Biologia Celular y Genetica de la Universidad de Malaga.)

The effect of the electric current on humans is almost independent on the frequency thereof up to 1000 Hz, which hinders the differentiation between a direct current source and an alternating current source. Below 1000 Hz there occur thermal phenomena, particularly electrolytic and capacitive phenomena. For frequencies higher than 350 KHz, the currents do not interfere with the nervous processes and only thermal energy can be produced. This explains the use of frequencies higher than 500 KHz (0.5 MHz) in electrosurgery. To these frequencies, the electrical conduction and organic absoption of the waves becomes more complex. There occur two mechanisms which produce heat: by the Joule effect, due to the electric resistance of the tissue to the current conduction and by absoption of the electromagnetic radiation, due to the moleclar structures. The water molecules - due to the electric and magnetic dipole structure - are stimulated by the incident electromagnetic waves and are entrained rapidly into rotation motion by the alternating electromagentic fields. The water molecules in rotation produce an effect of mechanical friction which is converted into heat. Together with the rapid polarity changes, the resistance and the rotation of the water molecules produce heat.

The produced resistance and heat inherently depend on the ion motion, impedance, treated tissue, reached depth, the phase of the electromagnetic wave which corresponds to the change of the applied radiofrequency field, change of supplied power, time of application and cooling conditions. One effect or the other will be of higher relevance if the pulses are increased. In the electro-surgery, both are important at frequencies of up to one MHz. For frequencies in the range of 1 to 3 MHz, the electromagnetic radiation effect is dominant.

At radiofrequencies equal to or lower than 10 - 15 MHz, the thermal effect is due to the Joule effect: the tissues oppose electric resistance upon the current passing and the radiofrequency energy is converted into heat. At more than 15 MHz, the radiofrequency energy produces its effect depending on the molecular friction. The radiofrequency emission may be classified into three groups:
a) - RF between 350 KHz - 1000 KHz
b) - RF between 1 MHz and 10 GHz. This type of radiofrequency penetrates the exposed tissue and causes its heating as a consequence of the energy absorption. The RF penetration depends on the electrical behaviour of the tissue, the tissue dimension as against the tissue production and tissue shape, geometry and orientation as against the radiation. The temperature rise may be of up to 11 °C
c) RF over 10 GHz: These emissions are absorbed by the skin surface and less energy reaches the lower tissue. Emissions having a power more than 1.0 KW/m² can cause dangerous effects on the health, such as skin burns or cataract ((16) "Medicina Preventive y Salud Publica", the 10th edition , Gonzalo Piedrola Gil, R.Galvez - Masson).

The study of the physical properties of the collagen revealed that this manifests piezoelectric effects: under the action of a force (literally) an electrostatic field with a specific orientation and polarity is produced.

This electrostatic field together with the microcurrents related thereto cause the ions and colloids within the local organic fluids to be redistributed towards specific locations (1) (Vibrational Medicine by Richard Berger, copyright 2005, Romanian edition at the ELIT Printing House, quotation page 16).

It is also possible the reversing of the phenomenon: inducing an electrical current within the collagen structure will determine the occurrence of mechanical displacements at a corresponding frequency which accelerates the transfer of substances between the dressing and wound, while reducing the suture and cicatrization period.

The use of these remarkable properties of collagen is the subject-matter of the present patent. The goal is to produce a new generation of dressings to make use of the electromagnetic effects of the interference between the living sound/wounded tissue, the biopolymeric dressing based on collagen (but also other compounds, for example according to the RO patent 126836 A0) and the bioresonant devices, electronic assemblies of the passive electromagnetic resonator type (for example, but not exclusively, according to US 6461375 B1). This combination invention benefits at least by the synergistic-piezoelectric effect caused by attaching, to a collagen-based dressing applied to a human or animal body wound, the passive electromagnetic resonator of a purposefully determined frequency - as a variable magnetic field source activated by the electromagnetic field of the human or animal body.

These variable electrical fields are the result of the interaction of the collagen structure with the variable magnetic component produced by the passive electromagnetic resonators, such as the ones present in the US patent 6,461,375 B1 , after activating the same by the electromagnetic field of the living human or animal body whereon it is positioned; the electromagetic field of the living beings is the result of the chemical reactions which maintain life in these bodies. The researches performed by Luigi Galvani - 1780, Kirlian Semyon - 1939 and others showed that an electromagnetic field is surrounding the living bodies.

In 1967 Bernard GRAD presented his descovery concerning the increase of the plant growth rate by the application of low intensity variable magnetic fields (1). (Vibrational Medicine by Richard Berger, page 629, ref. 5 - "The biological effects of the Laying on of Hands on Animals and Plants - Implications for Biology" in Parapsychology, Its Relation to Physics, Biology, Psychiatry and Psychology", G.Schmeidler edition (Metuchen, NJ: Scarecrow Press, 1967).

Studies published in 1972 by dr. Justa SMITH point out the acceleration of the kinetic activity of enzymes under the influence of the magnetic fields and the biofields capacity to repair the affected enzymes, by mechanisms similar to the variable magnetic fields ("The Influence on Enzyme Growth by the Laying on of Hands" in the Dimensions of Healing - A symposium, Los Altos, CA, The Academy of Parapsychology and Medicine, 1972) (1) (Vibrational Medicine by Richard Berger, copyright 2005, Romanian edition at the ELIT Printing House, quotation (1) page 162, page 164, ref. 8, page 629, Medicina Vibratjonala, ELIT Printing House).

In the prior art there exist numerous techniques for treating various pathological conditions by the external stimulation of specific points or areas on the body. Presently known kinds of stimulation are performed by apparatuses using a variety of techniques, amongst which we can mention the needles that are traditionally used in acupuncture, mechanical vibrations, electrical currents, electromagnetic radiations, magnets, light radiation (non-coherent visible light , laser light, ultraviolet light and infrared light,...), optical filtres etc.

Numerous stimulation points or areas are used that are distributed substantially over all of the surface of the body, and in this context, there can be mentioned: the points known for use in acupuncture, in reflexotherapy, in auriculo-medicine and in podological apparatuses. In order to illustrate the variety and condition of these teqhniques, varous patents that are representative in this field can be mentioned:
1) the French patent filed on Feb.10, 1975 under the number 75 04116 by the German company ELMATRON GmbH relating to ..Apparatuses for encouraging the healing of cells of human or animal organisms by means of electromagnetic pulses". That apparatus emits electromagnetic waves at a frequency that is not stated precisely (high frequerncies), but in the form of repetitive pulses at frequencies in the range 0 to 1000 Hz.
2) the German patent of October 29, 1975, extended to France on October 25, 1976 under the No. 76 32116 by Messerschmitt-BOLKOW-Blohm relating to ..Apparatus making use of the action of light for therapeutic treatments similar to acupuncture". This relates specifically to projecting low power laser radiation that is pulsed by means of a shutter at a frequency of 2 Hz to 20 Hz, thereby stimulating specified points (light spots that are about 1 mm in diameter) as determined in acupuncture and giving the same effects as are obtained using needles.
3) the French patent filed on November 24, 1976 under no. 76 32152 by Mr Pierre NOGIER, concerning "A method and apparatus for local stimulation by electromagnetic radiation". The radiation is produced by a beam of infrared radiation that is at least semicoherent, having a fundamental frequency of 73 Hz and including numerous harmonics, and illuminating selected points of the body (unspecified) and no details are given as to the effects obtained.
4) the French patent filed on February 15, 1979 under no. 7904486 by Mr.P. NOGIER, describing "A method and apparatus for magnetic treatment of living organisms". He describes a device of the Polaroid glass type that is suitable for polarizing light and that is interposed between a source of magnetic flux and the organism to be treated; unlike the polarized light, the the polarized magnetic flux then penetrates deep into the organism without losing its polarization.

The stimulation is sometimes continuous, but in most cases it is also pulsed or modulated by a low frequency signal.

Many of these apparatuses use electromagnetic emissions, particularly in the visible light or infrared spectrum.

Apparatuses of this kind are sometimes associated with filters (polarizing or coloured) for optimizing the positive effects of the declared source, of which some are used directly on the organism (laser, ultraviolet lamp, magnetic field etc.).

The filters are added in order to limit the radiation from the source and in order to allow only the active portion of the radiant flux to pass, said portion being thus consolidated.

Moreover, these apparatuses are applied to the points which are never specified clearly by the inventors, that generally observed the benefic effects on the organisms, but without the results or tests having been proved and described in their inventions. There are few techniques which do not make use of an external active source, such as the BRICOT's invention which uses polarization plates, Messrs MARIGAN and SOUVESTRE's invention using optical filters or those using magnets, such as Mr. Edouard LEBART's invention which combines magnets with the mechanical stimulation (French patent no. 2 687 075 of August 13,-1993).

The US patent 6,461 ,375 B1 presents a method for electromagnetic stimulation of the skin applied to a living organism and more particularly to the human organism, at certain points or certain areas that are determined by the pathology to be treated, or by the desired physiological modifications, by means of a resonant electronic device whose resonant frequency (ies) (hereinafter referred to as ..natural frequencies") are themselves determined by the looked-for effect expected from the stimulation.

Document EP 2127694 A1 discloses a device for the electrical treatment of a skin wound comprising a flexible substrate adapted for attaching to the skin wound and the surrounding skin and a matrix of electrodes, means for determining the presence of the wound and/or its perimeter and means for applying a voltage between the wound and its surrounding area. The document also discloses a method for electrical treatment of a skin wound comprising the steps: placing a matrix of electrodes attached to a flexible substrate on top of the skin wound, determining the presence of the wound and/or its perimeter, and charging at least one of the electrodes.

Document WO 2011/133539 A2 relates to a method for regulating conditions of mammalian skin of cosmetic nature such as wrinkles, sagging, dark under-eye circles etc by heating the collagen in the dermis of the skin via two or more electrodes delivering energy from a radiofrequency energy device, the electrodes contacting the stratum corneum via a gel composition.

Document WO 2013/074576 A2 is concerned with remodeling and down-sizing subcutaneous lipid-rich cells, body contouring and skin tightening by applying electromagnetic energy to the subcutaneous tissue from applicators placed adjacent to the skin but not touching the skin, such that the skin may be sufficiently cooled passively by ambient air or a stream of chilled or room temperature air. Such a method requires an external source of energy from an applicator containing one or more capacitive or inductive electrodes which apply an electromagnetic field on frequencies of typically 50 to 1500 Hz.

Document EP0227955 is concerned with providing a composite wound dressing comprising a layered arrangement of a semipermeable membrane and a biodegradable tissue interface, wherein both layers may be formed from synthetic collagen together with other components, and optionally a supporting and reinforcing permeable layer in between. Such a wound dressing is designed to simulate the function of normal skin such that it provides controlled water vapor permeability.

Document WO 2009/146441 A1 discloses a super-absorbent, reduced-pressure wound dressing including a super-absorbent bolster assembly, a sealing subsystem and a reduced-pressure subsystem that is obtained with the aid of a reduced-pressure source (a battery, generator or therapy unit).

Compared with known methods of stimulation by energy transfer carried out by generating a magnetic field or by emitting the electromagnetic waves by means of an active source, the method of the invention uses the resonant capability of an active or passive device to interact with the organism, mainly by absorbing the electromagnetic energy external to the apparatus.

The method consists in placing an electronic device in certain locations that are propitious for such a stimulation, so that the mentioned apparatus, under the influence of the magnetic field and the electromagneric radiation generated by the organism enters into resonance at its natural frequency (ies), which is determined depending on the effect looked for on the organism, and the apparatus is adjusted accordingly.

The invention uses electromagnetic coupling between the device of the invention and any structure of the organism capable of interacting with the apparatus by means of the mentioned coupling.

The resonance of the circuit as induced by the organism gives rise to a modification in the electromagnetic behaviour of the organism, and causes said organism to be stimulated.

The method therefore does not require direct contact with the organism and it can be implemented by means of a device that may possibly be completely passive, so that by the lack of the source of energy there is avoided any risk of exceeding the energy levels acceptable to the organism.

The advantage of such a method is that it enables a stimulation to be performed continuously, where the stimulation is effective while being inoffensive and can be applied to any point of the body including the sole of the foot, by means of devices of size that is very small, thereby enabling them to be worn continuously and discretely.

Furthermore, the desired resonant frequency associated with the pathology being treated, can easily be obtained in a device of the invention, as can all the harmonics of said frequency.

Because of its effective and prolonged action, this stimulation method has shown its effectiveness firstly as a palliative, and then in the longer term as a curative for various pathological conditions.

By way of example, mention can be made of spectacular controlled and measured results obtained on individuals suffering from balance troubles, following severe damage to the inner ear, e.g. to the perilymphatic fistulae.

Another application that has shown the effectiveness of the method and of the device is correcting troubles and affections of neuromuscular tonus concerning posture.

The apparatus of the invention for implementing the above-described method can itself be embodied by one or more electronic circuits exhibiting resonant behaviour, together with an element for electromagnetic coupling with the structures concerned of the organism.

Such a circuit has a mode of operation whereby, when it is at resonance, it absorbs energy from the organism which then behaves as a source of the said energy, said enery being amplified by the fact that this interchange takes place at certain special frequencies of the circuit, known as resonant frequencies. This phenomenon is well known and is used both in mechanics and in electronics.

The electromagnetic coupling is obtained by induction by means of a coil type element, or using a dipole type of device.

The calibration of these resonators to bioresonance frequencies that are in a harmonics type relationship with the response frequencies of the collagen structure, determines a synergistic effect on the wounded tissue, contributing to the rapid healing thereof. Our own researches led to the identification of a frequency band in the range of 0.5-5 MHz, which determines the occurrence of the synergistic effect of the structure formed of a passive resonator and a spongy collagen dressing.

The study of the medical techniques of acupuncture meant for the healing of wounds show that it is usual to place acupuncture needles around the contour of the deep wounds for the purpose of accelerating their healing.

There exists similarity between the two previously described techniques: they are based on the same phenomena, wherein the effects of the presence of the acupuncture needle are similar to those resulting due to the electromagnetic resonator presence.

Combining the said physical - magnetic, electrical and oscillatory - effects is ascertained to be at the basis of the previously described intervention technique.

It is an object of the present invention to provide a bioresonant dressing for the rapid healing of wounds of humans or animals as defined in claim 1.

It is a further object of the present invention to provide a process for producing said bioresonant dressing as defined in claim 2.

There are given hereinafter two/three embodiments of the invention in relation with the Figures 1 , 2, 3, 4 ...8, which represent:
Fig. 1 , the electrical diagram of the passive resonator, similar to Fig. 1 in the US patent 6,461 ,375 B ;
Fig. 2, view of the passive resonator;
Fig. 3, view of the bioresonant dressing with two rows of passive resonators;
Fig. 4, positioning the passive resonators in line/row depending on the orientation of the vector of the magnetic field of the coil;
Fig. 5, positioning the passive resonators onto the surface - version 5.1, depending on the orientation of the vector of the magnetic field of the coil;
Fig. 6, positioning the passive resonators onto the surface - version 5.2, depending on the orientation of the vector of the magnetic field of the coil;
Fig. 7, positioning the passive resonators onto the surface - version 5.3, depending on the orientation of the vector of the magnetic field of the coil;
Fig. 8, positioning the passive resonators onto the surface - version 5.4, depending on the orientation of the vector of the magnetic field of the coil.

The bioresonant dressing for the rapid healing of wounds, as claimed by the invention, consists of an assembly consisting of a biodegradable component presented as an absorbant composite - a spongy dressing (1) which consists of a biopolymeric dressing with three-dimensional conformation and porous microstructure consisting, for example, of collagen proteins, hyaluronic acid, growth factors and pharmaceutical factors with protective role and action stimulating the tissue regeneration, for example according to the RO patent 126836 A0 and a recoverable component presented as an assembly of elementary bioresonant electronic components, and passive resonators (3), respectively.

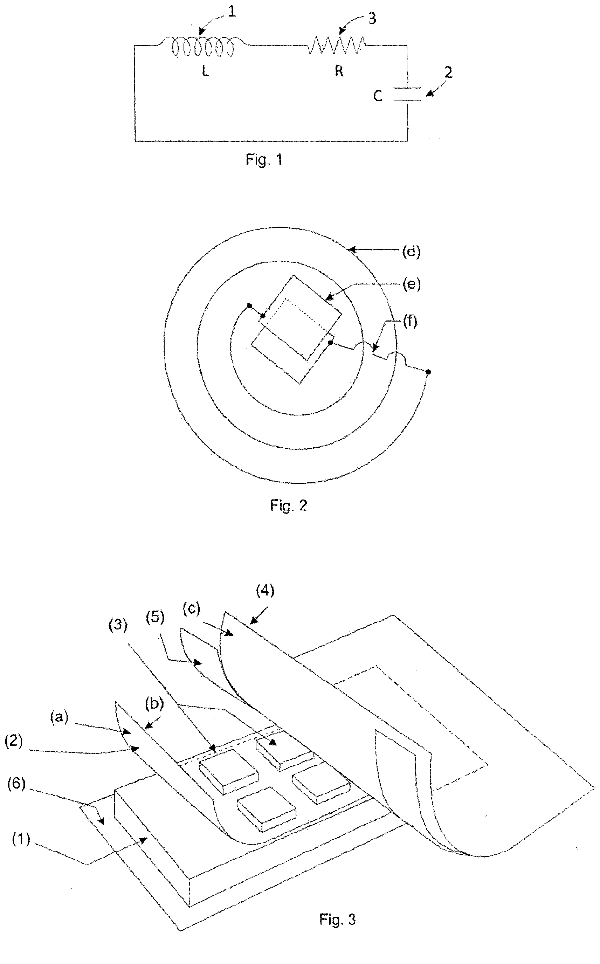

According to Fig. 1 , the resonant circuit which constitutes a passive resonator consists of an inductance 1 , L, a resistance 3, R and a capacitance 2, C having the following values for a resonant frequency of 1 MHz: L1 = 0,468microH, C1 =4nF, R1 = 1 Ohm.

For the resonance frequency of 3 MHz, the values of the components are: L2 =0,468microH, C2 =2nF, R2 = 1 Ohm.

For the resonance frequency of 5 MHz, the values of the components are: L3 = 1.04 microH, C3 = 1 nF, R3 = 1 Ohm.

According to Fig. 2, the flat coil (d) corresponds to inductance L, the capacitor (e) corresponds to the capacitance C and the resistor (f) corresponds to the resistance R.

According to Fig. 3, the bioresonant dressing consists of a spongy dressing (1) on top of which there is positioned a film (2) having the face (a) with adhesive which ensures its solidarization to the spongy dressing (1) and face (b) with the adhesive ensuring the fastening of the passive resonators (3) which may have various shapes in plane, such as for instance square or circular shape; on top of them there is assembled a film (4) provided with adhesive on the face (c) for a better fastening both to the film (2) and the passive resonators (3) as well as to the patient, by the adhesive zone protected up to the use by some tapes (5), the whole product being positioned on a film (6) having a surface sufficiently large in relation to the components positioned thereon, so as to allow its being made integral with the film (4) by means of the adhesive on the surface (c) and a protective enclosure to be formed.

The film (2) has the same dimensions in the plane as the spongy dressing (1) with a thickness in the range of 10 - 35 micron, being made up of an impervious material such as, for example, polyurethane or polyester and it is provided on the lower face and upper face with a layer (a) and (b), respectively, of adhesive material with a thickness of 25-50 micron, said layer consisting of acryl monomers, mixture of vinyl esters or other similar adhesives.

The film (4) has dimensions in the plane higher by 1.5-3 cm on the outline than the spongy dressing (1), has a thickness in the range of 10-35 microns and it is carried out of an impervious material such as polyurethane, polyesrer, polyvinyl chloride etc, being provided on the face oriented towards the body with an adhesive layer (c) with a thickness of 25-50 micron, for example, of acryl monomers, mixture of vinyl esters or other similar adhesives compatible to the human skin.

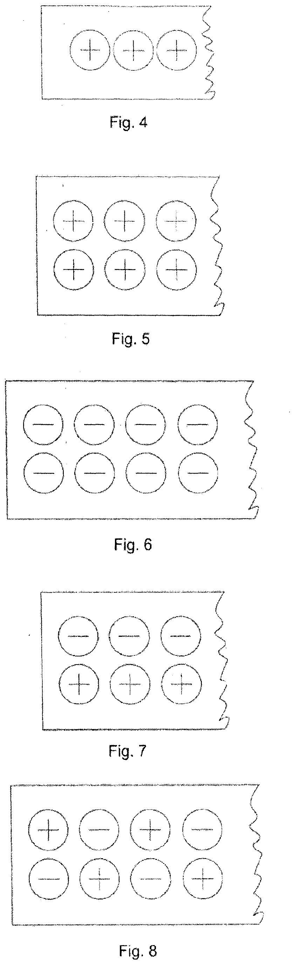

According to Fig. 4, 5, 6, 7 and 8 there are possible constructive and utilization versions of the passive resonator (3) by combinations of the orientation of the vector of the magnetic field produced by the coil (d) belonging to the passive resonator (3).

The process for obtaining a bioresonant dressing involves positioning on the film (6) and assembling in sequence a spongy dressing (1) made of a foam with absorbing properties carried out according to RO patent 126836 A0, with a film (2) provided with an adhesive on both sides (a) and (b), by overlapping and gently pressing, then the application of the final protection film (4) thereon and for securing to the body in the area of the wounds to be treated, provided with adhesive, similar to the one used on faces (a) and (b), on its face (c) oriented towards the spongy dressing (1), towards the film (2) and film (6), after having previously positioned on this face (c) of the film (4) the passive resonators (3) carried out, for example, as claimed in US patent 6,461 ,375 B1 - so that the orientation of the coils (d) thereof makes the sense of the magnetic field be in compliance with one of the options in claim 6, and the protective tapes (5), followed by protecting in the sterilization package formed by sticking films (4) and (6) and by sterilization with gamma rays or other similar processes.

The use of the bioresonant dressing carried out according to the above description is made by treating the wounds in humans and animals.

The advantages of the invention:
- By means of a modular device consisting of a consumable part and a recoverable part, there is ensured the rapid intervention for healing the wounds in humans and animals, with low costs due to recycling the recoverable component;
- The modular construction allows to carry out biodegradable components with various recipes, adaptable to the specific health condition of limited groups of patients - practically ensuring customization of intervention both with regard to the employed substances and with regard to the related radiofrequency field characteristics;
- It ensures the rapid healing of superficial or deep wounds by the cumulated and synergistic effect of the electronic, chemical, physical and physiological structures characteristics;
- It is applicable without strict restrictions for dimensions, employed frequencies or bioactive components;
- The treatments in which it is applied are available/self-applied treatments, on short term, with rapid and effortless application, with good results in aged patients.

## Claims

1. Bioresonant dressing for the rapid healing of wounds of humans or animals, consisting of an assembly comprising:
- a biodegradable component presented as an absorbent composite - a spongy dressing (1) which consists of a biopolymer dressing with three-dimensional conformation and porous microstructure consisting of collagen proteins, hyaluronic acid, growth factors and pharmaceutical factors with protective role and action meant to stimulate the tissue regeneration, and
- a recoverable component that can be recycled consisting of an assembly of elementary bioresonant electronic components and passive resonators (3), respectively, wherein said passive resonators (3) consist of elementary bioresonant electronic components having the resonance frequency in the range of 0.5 - 5 MHz, distributed linearly or on a surface and sustained by a flexible support dimensionally correlated with the first component and with the dimensions of the wound whose healing is intended, wherein said passive resonators (3) have an inductance (L), a resistance (R) and a capacitance (C), and wherein said one or more passive resonators (3) are distributed linearly/on rows or on a surface and sustained by an impervious film (2) provided with two adhesive layers (a) and (b), one on each of the faces, the layer (a) ensuring the fastening of the spongy dressing (1) with which it has equal dimensions, and the layer (b) ensuring the fastening of the passive resonators (3) on top of which there is applied a film (4) for final protection and for securing to the body in the area of the wounds to be treated, provided with adhesive on surface (c) oriented towards the film (2) and body, with dimensions increased by more than 1.5-3 cm as against the spongy dressing (1) so as to provide the additional surface required for fastening to the body.

2. Bioresonant dressing as claimed in claim 1 or 2, **characterized in that** the impervious film (2) has the same dimensions in plane as the spongy dressing (1) with a thickness ranging from 10 to 35 micron, being made of an impervious material such as polyurethane or polyester and it is provided on the lower face and on the upper face with a layer (a) and (b), respectively, of adhesive with the thickness of 25-50 micron, made up of acryl monomers, mixture of vinyl esters or other similar adhesives.

3. Bioresonant dressing according to any preceding claim, wherein the impervious film (4) has dimensions in plane higher by 1.5 - 3 cm on the contour than the spongy dressing (1), with a thickness in the range of 10-35 micron, being made of an impervious material such as, for example, polyurethane, polyester, polyvinyl chloride etc and it is provided on the face oriented towards the body with a layer (c) of adhesive with the thickness of 25-50 micron, for example acryl monomers, mixture of vinyl esters or other adhesives compatible with the human skin.

4. Process for producing a bioresonant dressing as claimed in claims 1 to 3, consisting in assembling in succession a spongy dressing (1) made of foam with absorbing properties with a film (2) provided with adhesive on both faces (a) and (b), by overlapping and gently pressing, then the application thereon of the film (4) for final protection and for fastening to the body in the area with the wounds to be treated, provided with an adhesive similar to the one used on faces (a) and (b) on the face (c) oriented towards the spongy dressing (1) and film (2), after having previously placed the passive resonators (3) onto the face (c) of film (4), so that the orientation of the vector of magnetic fields produced by the component coils of the bioresonant electronic elements is either all positive, all negative, alternating rows, alternating components in the same row or identical components in the same row, followed by protecting in the sterilization package and sterilizing with gamma rays.

## Patentansprüche

1. Bioresonanter Verband zur schnellen Heilung von Wunden bei Menschen oder Tieren, bestehend aus einer Anordnung umfassend:
- eine biologisch abbaubare Komponente, die als absorbierender Verbundwerkstoff vorliegt - und zwar ein schwammiger Verband (1), der aus einem Biopolymerverband mit dreidimensionaler Struktur und poröser Mikrostruktur bestehend aus Kollagenproteinen, Hyaluronsäure, Wachstumsfaktoren und pharmazeutische Faktoren mir schützender Funktion und Wirkung zur Stimulierung der Geweberegeneration besteht, und
- eine rückgewinnbare Komponente, die wiederverwertet werden kann, bestehend aus einer Anordnung einfacher bioresonanter elektronischer Komponenten bzw. passiver Resonatoren (3), wobei die passiven Resonatoren (3) aus einfachen bioresonanten elektronischen Komponenten mit der Resonanzfrequenz im Bereich von 0,5 - 5 MHz bestehen, die linear oder auf einer Oberfläche verteilt und von einem flexiblen Träger getragen werden, der mit der ersten Komponente und den Abmessungen der Wunde, deren Heilung beabsichtigt ist, dimensional korreliert ist, wobei die passiven Resonatoren (3) eine Induktivität (L), einen Widerstand (R) und eine Kapazität (C) aufweisen und wobei der eine oder die mehreren genannten passiven Resonatoren (3) linear/in Reihen oder auf einer Oberfläche verteilt sind und von einer undurchlässiger Folie (2) getragen werden, die mit zwei Klebeschichten (a) und (b), je eine auf jeder der Seiten, versehen ist, wobei die Schicht (a) die Befestigung des gleich großen, schwammigen Verbandes (1) sicherstellt und wobei die Schicht (b) die Befestigung der passiven Resonatoren (3) sicherstellt, auf die eine Folie (4) zum endgültigen Schutz und zur Befestigung am Körper im Bereich der zu behandelnden Wunden aufgebracht ist, welche auf der zur Folie (2) und zum Körper ausgerichtetenOberfläche (c) mit Klebstoff versehen ist, mit Abmessungen die gegenüber dem schwammigen Verband (1) um mehr als 1,5 - 3 cm vergrößert sind, um die zur Befestigung am Körper erforderliche zusätzliche Oberfläche bereitzustellen.

2. Bioresonanter Verband nach Anspruch 1 oder 2, **dadurch gekennzeichnet, dass** die undurchlässige Folie (2) die gleichen Abmessungen in der Ebene wie der schwammige Verband (1) mit einer Dicke im Bereich von 10 bis 35 Mikrometer aufweist, aus einem undurchlässigen Material wie Polyurethan oder Polyester hergestellt ist und auf der Unterseite und auf der Oberseite mit einer Klebstoffschicht (a) bzw. (b) mit einer Dicke von 25 - 50 Mikrometer versehen ist, die aus Acrylmonomeren, einer Mischung aus Vinylestern oder anderen ähnlichen Klebstoffen besteht.

3. Bioresonanter Verband nach einem der vorhergehenden Ansprüche, wobei die undurchlässige Folie (4) in der Ebene Abmessungen um 1,5 - 3 cm größer auf der Kontur als der schwammiger Verband (1), mit einer Dicke im Bereich von 10 bis 35 Mikrometer, die aus einem undurchlässigen Material wie zum Beispiel Polyurethan, Polyester, Polyvinylchlorid usw. hergestellt ist und auf der zum Körper gerichteten Fläche mit einer Klebeschicht (c) mit einer Dicke von 25 - 50 Mikrometer versehen ist, zum Beispiel aus Acrylmonomere, einer Mischung aus Vinylestern oder anderen mit der menschlichen Haut verträglichen Klebstoffen.

4. Verfahren zur Herstellung eines bioresonanten Verbandes nach den Ansprüchen von 1 bis 3, bestehend aus der aufeinanderfolgenden Montage eines schwammigen Verbandes (1) aus Schaumstoff mit absorbierenden Eigenschaften mit einer Folie (2), die auf beiden Seiten (a) und (b) mit Klebstoff versehen ist, durch Überlappen und sanftes Drücken und danach durch das Aufbringen der Folie (4) zum endgültigen Schutz und zur Befestigung am Körper im Bereich der zu behandelnden Wunden, mit einem Klebstoff, der demjenigen ähnelt, der auf den Flächen (a) und (b) verwendet wird, auf der Seite (c) die auf den schwammigen Verband (1) und die Folie (2) ausgerichtet ist, nach vorheriger Platzierung der passiven Resonatoren (3) auf der Fläche (c) der Folie (4), so dass die Ausrichtung des Vektors der Magnetfelder, die von den Komponentenspulen der elektronischen bioresonanten Elemente erzeugt werden, entweder alle positiven, alle negativen, abwechselnde Reihen, abwechselnde Komponenten in der gleichen Reihe oder identische Komponenten in der gleichen Reihe, gefolgt vom Schutz in der Entkeimungsverpackung und Entkeimung mit Gammastrahlen.

## Revendications

1. Pansement biorésonant pour la cicatrisation rapide de blessures humaines ou animales, constitué d'un ensemble comprenant:
- un composant biodégradable présenté sous forme de composite absorbant
- un pansement spongieux (1) consistant en un pansement en biopolymère à conformation tridimensionnelle et microstructure poreuse constituée de protéines de collagène, d'acide hyaluronique, de facteurs de croissance et de facteurs pharmaceutiques ayant un rôle protecteur et une action conçue pour stimuler la régénération des tissus, et
- un composant récupérable pouvant être recyclé, constitué d'un assemblage de composants électroniques biorésonants élémentaires et de résonateurs passifs (3), respectivement dans lequel lesdits résonateurs passifs (3) sont constitués de composants électroniques biorésonants élémentaires ayant une fréquence de résonance comprise entre 0,5 et 5 MHz, répartis linéairement ou sur une surface et maintenus par un support souple corrélé de manière dimensionnelle au premier composant et aux dimensions de la blessure dont la cicatrisation est destinée, dans lequel lesdits résonateurs passifs (3) ont une inductance (L), une résistance (R) et une capacitance (C), et dans lequel un ou plusieurs résonateurs passifs (3) sont répartis linéairement/sur des rangées ou sur une surface et sont maintenus par un film imperméable (2) muni de deux couches adhésives (a) et (b), une sur chacune des faces, la couche (a) assurant la fixation du pansement spongieux (1) avec lequel il a les mêmes dimensions, et la couche (b) assurant la fixation des résonateurs passifs (3) sur lesquels est appliqué un film (4) pour la protection finale et la fixation au corps dans le zone des blessures à traiter, munie d'adhésif sur la surface (c) orientée vers le film (2) et le corps, avec des dimensions augmentées de plus de 1,5-3 cm par rapport au pansement spongieux (1) afin de fournir la surface supplémentaire nécessaire pour la fixation au corps.

2. Pansement biorésonant selon la revendication 1 ou 2, **caractérisé en ce que** le film imperméable (2) a les mêmes dimensions dans le plan que le pansement spongieux (1) avec une épaisseur allant de 10 à 35 microns, étant constitué d'un matériau imperméable tel que du polyuréthane. ou du polyester et il est pourvu sur la face inférieure et sur la face supérieure d'une couche (a) et (b), respectivement, d'adhésif d'épaisseur comprise entre 25 et 50 microns, constituée de monomères acryliques, d'un mélange d'esters vinyliques ou autres adhésifs similaires.

3. Pansement biorésonant selon l'une quelconque des revendications précédentes, dans lequel le film imperméable (4) a des dimensions dans le plan supérieures de 1,5 à 3 cm sur le contour du pansement spongieux (1), avec une épaisseur dans la plage de 10 à 35 microns, en un matériau imperméable tel que, par exemple, du polyuréthane, du polyester, du chlorure de polyvinyle, etc. et il est pourvu sur la face orientée vers le corps d'une couche (c) d'adhésif d'une épaisseur de 25 à 50 microns, par exemple des monomères acryliques, mélange d'esters vinyliques ou d'autres adhésifs compatibles avec la peau humaine.

4. Procédé de fabrication d'un pansement biorésonant selon les revendications 1 à 3, consistant à assembler successivement un pansement spongieux (1) en mousse à propriétés absorbantes avec un film (2) pourvu d'un adhésif sur les deux faces (a) et (b), en se chevauchant et en appuyant doucement, puis l'application du film (4) sur celui-ci pour la protection finale et la fixation au corps dans la zone des blessures à traiter, muni d'un adhésif similaire à celui utilisé sur les faces (a) et (b) sur la face (c) orientée vers le pansement spongieux (1) et le film (2), après avoir préalablement placé les résonateurs passifs (3) sur la face (c) du film (4), de sorte que l'orientation du vecteur des champs magnétiques produits par les bobines de composant des éléments électroniques biorésonnants est soit tous positifs, tous négatifs, rangées alternantes, composants alternés dans la même rangée ou composants identiques dans la même rangée, suivis d'une protection dans l'emballage de stérilisation et d'une stérilisation avec des rayons gamma.
